# EUROPÄISCHE PATENTSCHRIFT

(11) **EP 1 295 616 B1**
(45) Veröffentlichungstag und Bekanntmachung des Hinweises auf die Patenterteilung: **17.12.2008**
(21) Anmeldenummer: 02020670.2
(22) Anmeldetag: 13.09.2002
(51) Int. Cl.: A61M 1/02

(54) **Blutspendegerät**
Blood donation apparatus
Appareil de don de sang

(30) Priorität: 25.09.2001 DE 10147208
(43) Veröffentlichungstag der Anmeldung: 26.03.2003
(73) Patentinhaber: Fresenius HemoCare Deutschland GmbH, 61352 Bad Homburg (DE)
(72) Erfinder: Kolb, Stefan, 61440 Oberursel (DE); Meisberger, Artur, 66606 Sankt Wendel (DE)
(74) Vertreter: Laufhütte, Dieter

(56) Entgegenhaltungen:
- DE-A- 3 737 304
- DE-U- 29 611 131
- FR-A- 2 574 540
- FR-A- 2 796 182
- US-A- 4 390 073
- US-A- 5 624 185

## Beschreibung

Die vorliegende Erfindung betrifft ein Blutspendegerät mit einer Aufnahme für ein Blutspendebehältnis sowie mit einem Bedienteil, an dem von dem Bediener benötigte Utensilien aufnehmbar sind und/oder mittels dessen Parameter des Spendevorgangs veränderbar, erfassbar und/oder abrufbar sind.

Ein Blutspendegerät mit einer eine Waage aufweisenden Wägeeinheit sowie mit einem Bedienteil, mittels dessen Parameter des Spendevorgangs veränderbar sind, ist beispielsweise aus der DE 37 37 304 C2 bekannt. Diese Druckschrift offenbart ein übliches Blutspendegerät, bei dem in einem Aufnahmebeutel das Spenderblut gesammelt wird. Der Aufnahmebeutel liegt auf einer als Aufnahme dienenden Waagschale einer elektronischen Wägeeinheit. Über eine durch ein Quetschventil laufende Schlauchleitung wird das Blut von dem Spender in den Aufnahmebeutel geführt. Das Quetschventil ist durch das Blutspendegerät aktivierbar, so daß der Blutspendevorgang bei Erreichen der entsprechenden Sollwerte, wie Blutmenge oder Blutspendedauer, unterbrochen bzw. der Blutstrom gedrosselt werden kann.

Die als Aufnahme für ein Blutspendebehältnis, insbesondere Blutbeutel, dienende Waagschale ragt oben aus dem Gehäuse des Blutspendegerätes heraus und ist mit der entsprechenden Wägeeinrichtung bzw. einer Schwenkeinrichtung über einen sich aus dem Gehäuse erstreckenden Schaft verbunden. Auf der Vorderseite des in der DE 37 37 304 C2 offenbarten Blutspendegerätes befindet sich eine Gewichtsanzeige, eine Niveaukontrolle sowie ein Tastenfeld. Mittels des Tastenfeldes kann das Gerät ein- und ausgeschaltet werden, der Spendevorgang unterbrochen werden, eine Wägung eingeleitet werden sowie das Gerät austariert werden.

Waagen mit Bedienfeldern sind in zahlreichen unterschiedlichen Anwendungsfällen bekannt, wie beispielsweise bei der Gewichtsermittlung von Lebensmitteln. Ein derartiges Gerät ist in der US 4,601,355 offenbart. Bei dem dort dargestellten Gerät ist ein separates Bedienteil vorgesehen, mit dessen Tastenfeld eingespeicherte Daten abrufbar sind und mittels dessen der Wägevorgang vereinfacht werden kann. Das Bedienteil ist bei Bedarf von dem Gehäuse der Waage abnehmbar.

Bei Blutspendegeräten der eingangs genannten Art fließt das Blut des Spenders aufgrund der Schwerkraft in den Aufnahmebeutel, der üblicherweise auf einer der Aufnahme dienenden Waagschale positioniert ist. Aus diesem Grunde werden vorbekannte Blutspendegeräte üblicherweise am Boden oder nur geringfügig erhöht aufgestellt.

Dies bringt den Nachteil mit sich, daß die Bedienung der Blutspendegeräte verhältnismäßig umständlich ist, da sich der Bediener des Gerätes stets bücken muß, um beispielsweise Daten abzulesen oder eine Änderung des Betriebes zu bewirken.

Aus US 5 624 185 ist ein Blutspendegerät mit einer Aufnahme für ein Blutspendebehältnis sowie mit einem Bedienteil bekannt, mittels dessen Parameter des Spendevorgangs veränderbar, erfassbar und abrufbar sind, wobei der Bedienteil gegenüber der Aufnahme des Blutspendegerätes erhöht angeordnet ist. Zudem wird vorgeschlagen, dass der Bedienteil eine optische Meldeeinrichtung aufweist, die dazu dient, Betriebszustände des Gerätes anzuzeigen. Das Bedienteil ist dabei an erhöhter Stelle in dem Gehäuse des Gerätes integriert, wobei die Oberseite des Gehäuses oberhalb des Bedienteils waagrecht ist.

Es ist daher die Aufgabe der vorliegenden Erfindung, ein Blutspendegerät der eingangs genannten Art dahingehend weiterzubilden, daß die Bedienung des Gerätes vereinfacht wird.

Diese Aufgabe wird ausgehend von einem Blutspendegerät gemäß dem Oberbegriff des Anspruchs 1 durch die Merkmale des kennzeichnenden Teils dieses Anspruchs gelöst. Zudem ist, wie bereits bekannt, der Bedienteil gegenüber der Aufnahme des Blutspendegerätes erhöht angeordnet. Dadurch wird ein häufiges Bükken beim Erfassen oder Ändern von Betriebsparametern überflüssig. Vielmehr genügt es, wenn der Bediener sich zu dem erhöht angeordneten Bedienteil orientiert, um hier die erforderlichen Maßnahmen zu ergreifen bzw. Anzeigen abzulesen. Die erhöhte Anordnung des Bedienteils bringt ferner den Vorteil mit sich, daß darin eventuell angeordnete Leuchten zur Anzeige bestimmter Betriebszustände besser erkennbar sind. Dies ist insbesondere deshalb von Bedeutung, da üblicherweise das Bedienpersonal nicht nur ein Gerät, sondern eine Vielzahl von Geräten gleichzeitig zu überwachen hat. Dies wird dadurch wesentlich vereinfacht, daß der Bedienteil erhöht angeordnet ist. Beispielsweise können Alarmzustände sehr viel schneller erkannt werden, als bei herkömmlichen Blutspendegeräten.

Üblicherweise steht die Aufnahme mit einer Meßeinrichtung, insbesondere einer Waage, zur Ermittlung der Menge des gespendeten Blutes in Verbindung. Die Meßeinrichtung kann gravimetrisch arbeiten, jedoch sind auch beliebige andere Meßverfahren, wie z.B. eine volumetrische Bestimmung der gespendeten Blutmenge einsetzbar.

In weiterer Ausgestaltung der vorliegenden Erfindung ist vorgesehen, daß der Bedienteil eine Tastatur aufweist, deren Tasten Betätigungseinrichtungen zum Ein- und Ausschalten des Gerätes, zum Unterbrechen des Blutspendevorgangs, zur Aktivierung und Deaktivierung einer Schwenkvorrichtung, zur Generierung einer Anzeige der Menge des gespendeten Blutes und/oder der noch verbleibenden Blutspendedauer sowie zum Austarieren darstellen. Auch kann vorgesehen sein, daß der Bedienteil nur die wichtigsten und häufig benutzten Tasten aufweist, während selten benutzte Tasten am Gehäuse des Blutspendegerätes angeordnet sein können.

Der Bedienteil kann eine Tastatur aufweisen, mittels derer das Gerät programmierbar ist. Beispielsweise kann vorgesehen sein, daß mittels der Tastatur die Dauer des Spendevorgangs und/oder die Menge des zu spendenden Blutes eingebbar ist. Auch für diese Vorgänge ist es erfindungsgemäß nicht erforderlich, daß sich der Bediener bückt, sondern die Eingabe dieser Daten ist in angenehmer Position dadurch möglich, daß der Bedienteil erhöht angeordnet ist.

Ferner kann vorgesehen sein, daß der Bedienteil ein Display aufweist, das dem Spender Informationen über den Verlauf des Spendevorgangs anzeigt. Bei am Boden angeordneten Anzeigen vorbekannter Geräte war es dem Spender nicht möglich, den Verlauf des Spendevorgangs, wie z.B. die bisher gespendete Blutmenge oder die noch verbleibende Zeit bis zum Ende des Spendevorgangs zu beobachten. Ferner kann an dem Bedienteil ein Bedienerdisplay vorgesehen sein, das dem Bediener Daten des Spendevorgangs anzeigt.

Der Bedienteil kann eine Meldeeinrichtung aufweisen, mittels derer Betriebszustände des Gerätes anzeigbar sind. Denkbar ist beispielsweise, daß Alarme oder auch das Ende der Spende angezeigt werden müssen. Aufgrund der erhöhten Anordnung des Bedienteils ist es auch aus größerer Entfernung für das Bedienpersonal ohne weiteres erkennbar, daß entweder beispielsweise eine Fehlfunktion vorliegt oder daß das Ende des Spendevorgangs erreicht ist. Damit schafft das erfindungsgemäße Blutspendegerät nicht nur einen erhöhten Komfort, sondern auch eine erhöhte Sicherheit, da Alarmzustände schnell erfassbar sind.

Die Meldeeinrichtung kann gegenüber dem Bedienteil derart ausziehbar sein, daß die Meldeeinrichtung gegenüber dem Bedienteil erhöht angeordnet werden kann. Auf diese Weise ist eine besonders gut einsehbare Position der Meldeeinrichtung realisierbar.

Gemäß einer weiteren Ausgestaltung der vorliegenden Erfindung ist vorgesehen, daß der Bedienteil eine Tastatur und/oder ein Display aufweist, das von dem Bedienteil abnehmbar ist. Auf diese Weise ist es möglich, daß der Bediener beispielsweise die Tastatur von dem Bedienteil abnimmt und direkt in die Hand nimmt, um die Eingabe bzw. das Erfassen von Betriebswerten noch weiter zu vereinfachen. Die Verbindung zwischen Bedienteil und Tastatur und/oder Display kann per Kabel oder auch per Infrarotübertragung etc. erfolgen.

Die von dem Bediener benötigten Utensilien können einen Barcodeleser sowie eine Schweißzange umfassen, wobei an dem Bedienteil Halterungen zur Aufnahme dieser Geräte vorgesehen sind. Der Barcodeleser wird beispielsweise benötigt, um den Blutspendebeutel bzw. einen daran angeordneten Konnektor genau zu identifizieren. Die Schweißzange wird benötigt, um am Ende des Spendevorgangs Schläuche zu verschließen.

In bevorzugter Ausgestaltung der vorliegenden Erfindung ist vorgesehen, daß der Bedienteil an einer sich von einem Gehäuse des Blutspendegerätes vertikal erstreckenden oder schräg verlaufenden Stütze angeordnet ist.

Besonders vorteilhaft ist es, wenn die Stütze gegenüber dem Gehäuse verschwenkbar angeordnet ist. Auf diese Weise ist eine geeignete Positionierung des Bedienteils möglich.

Weiterhin kann vorgesehen sein, daß der Bedienteil gegenüber der Stütze bewegbar angeordnet ist. Hierbei ist denkbar, daß beispielsweise das dem Spender zugewandte Display verschwenkt werden kann, so daß dieses optimal einsehbar ist. Ferner ist denkbar, daß die Meldeeinrichtung so verschwenkt oder verdreht werden kann, daß diese von dem Bedienpersonal besonders gut eingesehen werden kann, so daß insbesondere ein Alarmzustand schnell erfaßt werden kann.

In weiterer Ausgestaltung ist vorgesehen, daß die Stütze in ihrer Länge veränderbar ausgeführt ist. Auf diese Weise ist erreichbar, daß sich der Bedienteil für unterschiedlich große Personen stets in optimaler Höhe befindet.

Die Stütze kann von dem Gehäuse abnehmbar ausgeführt sein. Die Stütze ist gemäß dieser Ausführungsform als gesamtes Teil abnehmbar und kann je nach Bedarf und Komfortbedürfnis an dem Gehäuse des Blutspendegerätes angebracht werden.

Besonders vorteilhaft ist es, wenn die Stütze mit dem Gehäuse durch eine Steckverbindung verbindbar ist, mittels derer sowohl die mechanische Verbindung als auch die elektronische Kontaktierung herstellbar ist. Auf diese Weise kann durch ein einfaches Einstecken der Stütze in das Gehäuse bzw. eine entsprechende Aufnahme eine mechanische Fixierung der Stütze und gleichzeitig auch die Herstellung der erforderlichen elektrischen bzw. elektronischen Kontakte realisiert werden.

Weitere Einzelheiten und Vorteile der vorliegenden Erfindung werden anhand eines in der Zeichnung dargestellten Ausführungsbeispiels näher erläutert. Es zeigen:
- Fig. 1:: eine perspektivische Ansicht eines Blutspendegerätes und
- Fig. 2:: eine perspektivische Darstellung des erfindungsemäßen Bedienteils mit Spenderdisplay, Tastatur und ausziehbarer Meldeeinheit.

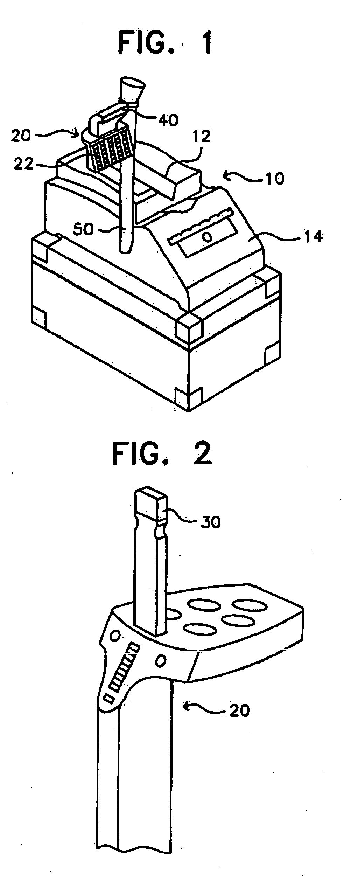

Figur 1 zeigt in perspektivischer Ansicht das Blutspendegerät 10, das geringfügig erhöht auf einem am Boden positionierten Träger angeordnet ist. Das Blutspendegerät 10 weist eine Waage 12 auf, in deren Schale zum Blutspendebetrieb ein Aufnahmebeutel eingelegt wird. Das Blutspendegerät 10 weist das Gehäuse 14 auf.

Die Waage 12 ist so angeordnet, daß sich ein Gefälle zwischen dem Blutspender und dem Aufnahmebeutel ergibt.

Von dem Gehäuse erstreckt sich vertikal die Stütze 50, an der der Bedienteil 20 angeordnet ist. Die Stütze muß sich nicht zwangsläufig vertikal erstrecken, sondern kann auch schräg nach oben verlaufen. Der Bedienteil 20 weist eine Halterung für einen Barcodeleser 40 sowie eine Tastatur 22 auf. Über die Tastatur 22 kann der Bediener nun in geeigneter Höhe Parameter des Spendevorgangs verändern, d.h. beispielsweise das Gerät aus- oder einschalten, den Spendevorgang unterbrechen oder auch Anzeigen generieren oder Daten ausdrucken oder beispielsweise auch das Gerät programmieren. Das Blutspendegerät weist zu diesem Zweck eine Steuereinheit sowie einen Speicher auf, über die der entsprechende Ablauf des Blutspendevorgangs geregelt wird.

Weiterhin kann über die Tastatur 22 die Waage austariert werden oder auch eine Schwenkvorrichtung aktiviert bzw. deaktiviert werden.

Figur 2 zeigt in einer perspektivischen Darstellung den Bedienteil 20 mit Spenderdisplay und Tastatur sowie mit der ausziehbaren optischen Meideeinrichtung 30. Diese weist Signalleuchten bzw. ein entsprechendes Display auf, durch das optisch bestimmte Betriebszustände des Gerätes, wie beispielsweise ein Alarm oder auch das Ende des Blutspendevorgangs deutlich angezeigt werden können. Aufgrund der erhöhten Anordnung der Meldeeinrichtung 30 ist für das Bedienpersonal besonders gut erkennbar, ob derartige Betriebszustände vorliegen.

Die Meldeeinrichtung 30 ist gegenüber dem Bedienteil 20 austeleskopierbar und in Figur 2 in diesem Zustand dargestellt. Wird diese nicht benötigt, wird die Meldeeinrichtung 30 eingeschoben.

Insgesamt ergibt sich durch das erfindungsgemäße Blutspendegerät die Möglichkeit, den Bedienungskomfort des Blutspendegerätes durch die erhöhte Position des Bedienteils erheblich zu verbessern und darüber hinaus die Sicherheit für den Blutspender dadurch zu erhöhen, daß beispielsweise optische Einrichtungen ebenfalls an erhöhter Position vorgesehen sein können, die dem Bedienpersonal sofort und an günstig einsehbarer Stelle einen fehlerhaften Betriebszustand oder auch das Ende des Spendevorgangs anzeigen können.

## Patentansprüche

1. Blutspendegerät (10) mit einer Aufnahme für ein Blutspendebehältnis sowie mit einem Bedienteil (20), an dem von dem Bediener benötigte Utensilien aufnehmbar sind und/oder mittels dessen Parameter des Spendevorgangs veränderbar, erfassbar und/oder abrufbar sind, wobei der Bedienteil (20) gegenüber der Aufnahme des Blutspendegerätes (10) erhöht angeordnet ist, wobei der Bedienteil (20) eine optische Meldeeinrichtung (30) aufweist, die dazu dient, Betriebszustände des Gerätes (10) anzuzeigen,
**dadurch gekennzeichnet,**
**dass** die optische Meldeeinrichtung (30) gegenüber dem Bedienteil (20) ausziehbar ist, so dass die optische Meldeeinrichtung (20) gegenüber dem Bedienteil (20) durch entsprechendes Ausziehen erhöht angeordnet werden kann.

2. Blutspendegerät (10) nach Anspruch 1, **dadurch gekennzeichnet, daß** der Bedienteil (20) eine Tastatur (22) aufweist, deren Tasten Betätigungseinrichtungen zum Ein- und Ausschalten des Gerätes (10), zum Unterbrechen des Blutspendevorgangs, zur Aktivierung und Deaktivierung einer Schwenkvorrichtung, zur Generierung einer Anzeige der Menge des gespendeten Blutes und/oder der noch verbleibenden Blutspendedauer sowie zum Austarieren darstellen.

3. Blutspendegerät (10) nach Anspruch 1 oder 2, **dadurch gekennzeichnet, daß** der Bedienteil (20) eine Tastatur (22) aufweist, mittels derer das Gerät (10) programmierbar ist.

4. Blutspendegerät (10) nach Anspruch 3, **dadurch gekennzeichnet, daß** mittels der Tastatur (22) die Dauer des Spendevorgangs und/oder die Menge des zu spendenden Blutes eingebbar ist.

5. Blutspendegerät (10) nach einem der Ansprüche 1 bis 4, **dadurch gekennzeichnet, daß** der Bedienteil (20) ein Display aufweist, das dem Spender Informationen über den Verlauf des Spendevorgangs anzeigt.

6. Blutspendegerät (10) nach einem der Ansprüche 1 bis 5, **dadurch gekennzeichnet, daß** der Bedienteil (20) eine Tastatur (22) und/oder ein Display aufweist, das von dem Bedienteil (20) abnehmbar ist.

7. Blutspendegerät (10) nach einem der Ansprüche 1 bis 6, **dadurch gekennzeichnet, daß** der Bedienteil (20) an einer sich von einem Gehäuse (14) des Blutspendegerätes vertikal erstreckenden oder schräg verlaufenden Stütze (50) angeordnet ist.

8. Blutspendegerät (10) nach Anspruch 7, **dadurch gekennzeichnet, daß** die Stütze (50) gegenüber dem Gehäuse (14) verschwenkbar angeordnet ist.

9. Blutspendegerät (10) nach Anspruch 7 oder 8, **dadurch gekennzeichnet, daß** der Bedienteil (20) gegenüber der Stütze (50) bewegbar angeordnet ist.

10. Blutspendegerät (10) nach einem der Ansprüche 7 bis 9, **dadurch gekennzeichnet, daß** die Stütze (50) in ihrer Länge veränderbar ausgeführt ist.

11. Blutspendegerät (10) nach einem der Ansprüche 7 bis 10, **dadurch gekennzeichnet, daß** die Stütze (50) von dem Gehäuse (14) abnehmbar ausgeführt ist.

12. Blutspendegerät (10) nach Anspruch 11, **dadurch gekennzeichnet, daß** die Stütze (50) mit dem Gehäuse (14) durch eine Steckverbindung verbindbar ist, mittels der sowohl die mechanische Verbindung als auch die elektronische Kontaktierung herstellbar ist.

## Claims

1. A blood donor device (10) comprising a receiving means for a blood donor container and an operating portion (20) at which instruments required by the operator can be received and/or by means of which parameters of the donor operation can be altered, detected and/or retrieved, wherein the operating portion (20) is arranged in elevated relationship with the receiving means of the blood donor device (10), wherein the operating portion (20) has an optical signalling device (30) which serves to display operating states of the device (10),
**characterised in that**
the optical signalling device (30) is extendable with respect to the operating portion (20) so that the optical signalling device (30) can be arranged in elevated relationship with the operating portion (20) by suitable extension.

2. A blood donor device (10) according to claim 1 **characterised in that** the operating portion (20) has a keyboard (22), the keys of which represent actuating devices for switching the device (10) on and off, for interrupting the blood donor operation, for activating and deactivating a pivoting device, for generating a display of the amount of blood donated and/or the blood donor time still remaining and for calibration.

3. A blood donor device (10) according to claim 1 or claim 2 **characterised in that** the operating portion (20) has a keyboard (22), by means of which the device (10) is programmable.

4. A blood donor device (10) according to claim 3 **characterised in that** the duration of the donor operation and/or the amount of blood to be donated can be inputted by means of the keyboard (22).

5. A blood donor device (10) according to one of claims 1 to 4 **characterised in that** the operating portion (20) has a display which displays to the donor information about the course of the donor operation.

6. A blood donor device (10) according to one of claims 1 to 5 **characterised in that** the operating portion (20) has a keyboard (22) and/or a display which is removable from the operating portion (20).

7. A blood donor device (10) according to one of claims 1 to 6 **characterised in that** the operating portion (20) is arranged on a support (50) extending inclinedly or vertically from a housing (14) of the blood donor device.

8. A blood donor device (10) according to claim 7 **characterised in that** the support (50) is arranged pivotably with respect to the housing (14).

9. A blood donor device (10) according to claim 7 or claim 8 **characterised in that** the operating portion (20) is arranged movably with respect to the support (50).

10. A blood donor device (10) according to one of claims 7 to 9 **characterised in that** the support (50) is variable in its length.

11. A blood donor device (10) according to one of claims 7 to 10 **characterised in that** the support (50) is adapted to be removable from the housing (14).

12. A blood donor device (10) according to claim 11 **characterised in that** the support (50) can be connected to the housing (14) by a plug connection, by means of which it is possible to make both the mechanical connection and also electronic contacting.

## Revendications

1. Appareil de don de sang (10) avec un logement pour un contenant de don de sang et avec une partie d'actionnement (20) à laquelle peuvent être reçus des ustensiles requis par l'utilisateur et/ou au moyen de laquelle des paramètres de l'opération de don peuvent être modifiés, détectés et/ou appelés, où la partie d'actionnement (20) est disposée d'une manière surélevée par rapport au logement de l'appareil de don de sang (10), où la partie d'actionnement (20) présente une installation de signalisation optique (30), qui sert à indiquer des états de fonctionnement de l'appareil (10),
**caractérisé**
**en ce que** l'installation de signalisation optique (30) peut être extraite par rapport à la partie d'actionnement (20) de sorte que l'installation de signalisation optique (20) peut être disposée d'une manière surélevée par rapport à la partie d'actionnement (20) par une extraction correspondante.

2. Appareil de don de sang (10) selon la revendication 1, **caractérisé en ce que** la partie d'actionnement (20) présente un clavier (22) dont les touches représentent des installations d'actionnement pour la mise en et hors service de l'appareil (10), pour l'interruption de l'opération de don de sang, pour l'activation et la désactivation d'un dispositif de pivotement, pour la génération d'un affichage de la quantité du sang donné et/ou de la durée de don de sang encore restante ainsi que pour la remise à zéro.

3. Appareil de don de sang (10) selon la revendication 1 ou 2, **caractérisé en ce que** la partie d'actionnement (20) présente un clavier (22) au moyen duquel l'appareil (10) peut être programmé.

4. Appareil de don de sang (10) selon la revendication 3, **caractérisé en ce qu'**au moyen du clavier (22), la durée de l'opération de don et/ou la quantité du sang à donner peuvent être entrées.

5. Appareil de don de sang (10) selon l'une des revendications 1 à 4, **caractérisé en ce que** la partie d'actionnement (20) présente un affichage qui fournit au donneur des informations se rapportant au déroulement de l'opération de don.

6. Appareil de don de sang (10) selon l'une des revendications 1 à 5, **caractérisé en ce que** la partie d'actionnement (20) présente un clavier (22) et/ou un affichage qui peut être retiré de la partie d'actionnement (20).

7. Appareil de don de sang (10) selon l'une des revendications 1 à 6, **caractérisé en ce que** la partie d'actionnement (20) est disposée sur un support (50) s'étendant verticalement ou s'étendant en biais à partir d'un boîtier (14) de l'appareil de don de sang.

8. Appareil de don de sang (10) selon la revendication 7, **caractérisé en ce que** le support (50) est disposé d'une manière pivotante par rapport au boîtier (14).

9. Appareil de don de sang (10) selon la revendication 7 ou 8, **caractérisé en ce que** la partie d'actionnement (20) est disposée d'une manière mobile par rapport au support (50).

10. Appareil de don de sang (10) selon l'une des revendications 7 à 9, **caractérisé en ce que** le support (50) est réalisé d'une manière modifiable en longueur.

11. Appareil de don de sang (10) selon l'une des revendications 7 à 10, **caractérisé en ce que** le support (50) est réalisé d'une manière retirable du boîtier (14).

12. Appareil de don de sang (10) selon la revendication 11, **caractérisé en ce que** le support (50) peut être relié au boîtier (14) par une liaison à emboîtement au moyen de laquelle peut être réalisée à la fois la liaison mécanique et aussi la mise en contact électronique.
